(19) Europäisches Patentamt / European Patent Office / Office européen des brevets

(11) **EP 4 040 219 A1**

(12) **EUROPEAN PATENT APPLICATION**
published in accordance with Art. 153(4) EPC

(43) Date of publication:
**10.08.2022 Bulletin 2022/32**

(21) Application number: **20872909.5**

(22) Date of filing: **27.07.2020**

(51) International Patent Classification (IPC):
**G02C 7/02** (2006.01)   **A61B 3/08** (2006.01)
**G02B 27/02** (2006.01)

(52) Cooperative Patent Classification (CPC):
**A61B 3/08; G02B 27/02; G02C 7/02**

(86) International application number:
**PCT/JP2020/028743**

(87) International publication number:
**WO 2021/065170 (08.04.2021 Gazette 2021/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.09.2019   JP 2019180310**

(71) Applicant: **Hoya Lens Thailand Ltd.**
**Pathumthani 12130 (TH)**

(72) Inventor: **QI Hua**
**Tokyo 160-8347 (JP)**

(74) Representative: **Betten & Resch**
**Patent- und Rechtsanwälte PartGmbB**
**Maximiliansplatz 14**
**80333 München (DE)**

(54) **BINOCULAR FUNCTION MEASURING METHOD, BINOCULAR FUNCTION MEASURING PROGRAM, DESIGN METHOD FOR SPECTACLE LENS, MANUFACTURING METHOD FOR SPECTACLE LENS, AND BINOCULAR FUNCTION MEASURING SYSTEM**

(57) Provided is a binocular visual function measurement method including a visual target presentation step of presenting a right eye image to be viewed by the right eye of a measurement subject and a left eye image to be viewed by the left eye of the measurement subject to the subject in a space where real space information is blocked out; a presentation control step of changing positions where the right eye image and the left eye image are presented, relative to each other; a timing detection step of detecting a timing at which the measurement subject is unable to fuse the right eye image and the left eye image when the presentation positions are changed; and a parameter value calculation step of calculating a predetermined parameter value regarding a binocular visual function of the measurement subject based on a relationship between the relative positions of the right eye image and the left eye image when the timing is detected.

**FIG. 3**

**Description**

Technical Field

The present invention relates to a binocular visual function measurement method, a binocular visual function measurement program, an eyeglass lens designing method, an eyeglass lens manufacturing method, and a binocular visual function measurement system.

Background Art

Eyeglass wearers may be subjected to binocular visual function examinations to measure convergence and divergence ranges, for example. There are individual differences in convergence and divergence ranges, and it is very important to measure the convergence and divergence ranges in order to understand the functions of the eyes in near vision in designing of eyeglass lenses.

With regard to the binocular visual functions represented by the convergence and divergence ranges and the like, Patent Document 1 discloses that the binocular visual function is measured by presenting left and right parallax images using a stationary three-dimensional compatible video monitor, moving the positions where they are presented, relative to each other, and detecting the timing at which images cannot be fused, for example.

Citation List

Patent Documents

Patent Document 1: JP-2012-95693A

Summary of Invention

Technical Problem

In the binocular visual function measurement method disclosed in Patent Document 1, the binocular visual function is measured using a stationary three-dimensional compatible video monitor. Thus, the position of the head of a measurement subject is not fixed with respect to left and right parallax images, and thus there is a risk that an error may occur in the median plane depending on the orientation of the head during measurement. Furthermore, by placing the stationary three-dimensional compatible video monitor in a real environment, in addition to parallax information to be displayed, the measurement subject simultaneously acquires information (real space information) that gives a sense of depth and perspective from the outside world, which may result in the convergence and divergence that occur together with normal accommodation. Furthermore, because the stationary three-dimensional compatible video monitor is used, the size of the required system configuration is increased, and thus it cannot be said that the binocular visual function can be easily measured.

The present invention aims to provide a technique by which the binocular visual function of a measurement subject can be very easily measured with high accuracy.

Solution to Problem

The present invention was made to achieve the above-described aim.

A first aspect of the present invention is directed to a binocular visual function measurement method, the method including:

a visual target presentation step of presenting a right eye image to be viewed by the right eye of a measurement subject and a left eye image to be viewed by the left eye of the measurement subject to the subject in a space where real space information is blocked out;
a presentation control step of changing positions where the right eye image and the left eye image are presented, relative to each other;
a timing detection step of detecting a timing at which the measurement subject is unable to fuse the right eye image and the left eye image when the presentation positions are changed; and
a parameter value calculation step of calculating a predetermined parameter value regarding a binocular visual function of the measurement subject based on a relationship between the relative positions of the right eye image and the left eye image when the timing is detected.

A second aspect of the present invention is directed to the binocular visual function measurement method according to the first aspect,
in which the right eye image and the left eye image are presented using a head mounted display to be worn by the measurement subject.

A third aspect of the present invention is directed to the binocular visual function measurement method according to the first or second aspect,
in which the predetermined parameter value is a value for specifying a convergence range of the measurement subject.

A fourth aspect of the present invention is directed to the binocular visual function measurement method according to any one of the first to third aspects, the method including

a tracking ability determination step of determining a level of the ability of an eye of the measurement subject to track a change in positions of the presented images,
in which, in the presentation control step, the speed of a change in relative positions of the right eye image and the left eye image is determined based on a result of the determination performed in the tracking ability determination step.

A fifth aspect of the present invention is directed to the binocular visual function measurement method according to any one of the first to fourth aspects,
in which the right eye image and the left eye image are constituted by figures having the same shape and the same size.

A sixth aspect of the present invention is directed to the binocular visual function measurement method according to any one of the first to fifth aspects, the method including
a visual range setting step of setting a visual range of the measurement subject with respect to the right eye image and the left eye image.

A seventh aspect of the present invention is directed to a binocular visual function measurement program for causing a computer to execute the binocular visual function measurement method according to any one of the first to sixth aspects.

An eighth aspect of the present invention is directed to an eyeglass lens designing method, the method including:

a step of measuring the binocular visual function of the measurement subject using the binocular visual function measurement method according to any one of the first to sixth aspects; and
a step of determining an optical design value of the eyeglass lens based on a result of the measurement of the binocular visual function.

A nineth aspect of the present invention is directed to an eyeglass lens manufacturing method, the method including:

a step of designing an eyeglass lens using the eyeglass lens designing method according to the eighth aspect; and
a step of manufacturing the eyeglass lens according to a result of designing the eyeglass lens.

A tenth aspect of the present invention is directed to a binocular visual function measurement system, the system including:

a visual target presentation unit configured to present a right eye image to be viewed by the right eye of a measurement subject and a left eye image to be viewed by the left eye of the measurement subject to the subject in a space where real space information is blocked out;
a presentation control unit configured to change positions where the right eye image and the left eye image are presented, relative to each other;
a timing detection unit configured to detect a timing at which the measurement subject is unable to fuse the right eye image and the left eye image when the presentation positions are changed; and
a parameter value calculation unit configured to calculate a predetermined parameter value regarding a binocular visual function of the measurement subject based on a relationship between the relative positions of the right eye image and the left eye image when the timing is detected.

An eleventh aspect of the present invention is directed to the binocular visual function measurement system according to the tenth aspect,
in which the visual target presentation unit is configured using a head mounted display to be worn by the measurement subject.

Advantageous Effects of Invention

According to the present invention, the binocular visual function of a measurement subject can be very easily measured with high accuracy.

Brief Description of Drawings

FIG. 1 is a block diagram showing a configuration of an eyeglass lens manufacturing system for realizing an eyeglass lens manufacturing method according to an embodiment of the present invention.

FIG. 2 is a diagram showing an overview of the binocular visual function measurement system according to the embodiment of the present invention.

FIG. 3 is a block diagram showing a configuration of the binocular visual function measurement system according to the embodiment of the present invention.

FIG. 4 is a diagram showing a flowchart of processing executed in a convergence range measurement mode by a binocular visual function measurement program according to the embodiment of the present invention.

FIG. 5 shows transition diagrams of images displayed on a display screen during execution of the convergence range measurement mode according to the embodiment of the present invention.

FIG. 6 is a diagram showing a flowchart of processing executed in a left-right eye vertical divergence allowable value measurement mode by the binocular visual function measurement program according to the embodiment of the present invention.

FIG. 7 shows transition diagrams of images displayed on a display screen during execution of the left-right eye vertical divergence allowable value measurement mode according to the embodiment of the present invention.

FIG. 8 is a diagram showing a flowchart of processing executed in a first unequal magnification allowable value measurement mode by the binocular visual function measurement program according to the embodiment of the present invention.

FIG. 9 shows transition diagrams of images displayed on the display screen during execution of the first unequal magnification allowable value measurement mode according to the embodiment of the present invention.

FIG. 10 is a diagram showing a flowchart of processing executed in a second unequal magnification allowable value measurement mode by the binocular visual function measurement program according to the embodiment of the present invention.

FIG. 11 shows transition diagrams of images displayed on the display screen during execution of the second unequal magnification allowable value measurement mode according to the embodiment of the present invention.

FIG. 12 shows a diagram for describing Listing's law.

FIG. 13 is a diagram illustrating the line-of-sight direction of the left and right eyes in the case of binocular vision.

FIG. 14 is a diagram showing a flowchart of processing executed in a left-right eye rotation parallax allowable value measurement mode by the binocular visual function measurement program according to the embodiment of the present invention.

FIG. 15 shows transition diagrams of images displayed on the display screen during execution of the left-right eye rotation parallax allowable value measurement mode according to the embodiment of the present invention.

FIG. 16 shows an example of the display of images in a first composite measurement mode.

FIG. 17 shows an example of the display of images in a second composite measurement mode.

FIG. 18 shows an example of the display of images in a third composite measurement mode.

FIG. 19 shows an example of the display of images in a measurement mode considering a lateral view.

Description of Embodiments

Hereinafter, an embodiment of the present invention will be described with reference to the drawings.

<Overview of Embodiment

First, an overview of this embodiment will be described.

In the present embodiment, a head mounted display (abbreviated as "HMD" hereinafter) to be worn by a measurement subject is used, and a pair of images with parallax are respectively presented to the left and right eyes of the measurement subject. The binocular visual function of the measurement subject is measured by determining whether the images are fused (identified) when the given parallax is changed. The binocular visual function is measured by calculating a predetermined parameter value for the binocular visual function.

Examples of the predetermined parameter value include values for specifying a convergence range of the measurement subject. A "convergence range" herein refers to the difference in angle between the convergence limit and the divergence limit. In the following description, a case where the convergence range is measured will mainly be described as an example. The predetermined parameter value is not limited to the values in the convergence range, and may be another parameter value such as the left-right eye vertical divergence allowable value, the first unequal magnification allowable value, the second unequal magnification allowable value, or the left-right eye rotation parallax allowable value, which will be described later.

In the present embodiment, because the HMD worn by the measurement subject is used in measurement of the binocular visual function thereof, the parallax images can be presented to the measurement subject in a space where real space information is blocked out. That is, the parallax images are presented in front of the eyes of the measurement subject in a state where the outside world is screened out, and thus, the measurement subject does not acquire information (real space information) that gives a sense of depth and perspective from the outside world, in addition to the presented parallax images.

Furthermore, by mounting an HMD on the measurement subject, the parallax images can be accurately positioned regardless of the direction of the face of the measurement subject.

Also, the visual range to the parallax images (the physical distance between the images and the subject who sees the images) is also kept constant, and thus it is possible to measure the binocular visual function while keeping the accommodation function (focusing function) of the eyes of the measurement subject constant.

Therefore, according to the present embodiment, it is possible to perform composite measurements in the same measurement environment without taking the posture and the position of the measurement subject into consideration. Also, by presenting only a pair of parallax images using the HMD, it is possible to measure the capability relating to a pure convergence range without giving a sense of depth, and to reduce the influence of accommodative convergence. "Accommodative convergence" here refers to convergence (convergence and divergence movement) that occurs simultaneously with accommodation in the near triad that occurs when looking at a near position.

That is, according to the present embodiment, it is possible to very easily measure the convergence range while eliminating three-dimensional perception by the measurement subject. If the convergence range of the measurement subject can be very easily measured with high accuracy in this manner, an eyeglass lens suitable for the measurement subject can be provided by using the results of measurements as one of the parameters for designing the eyeglass lens.

For example, a measurement subject who has been found to have weak motor fusion based on the results of measurements is likely to complain of diplopia in which images are unlikely to fuse under strong binocular separation. Therefore, it is possible to provide an eyeglass lens with a lens design that compensates for weak motor fusion by inserting a prism to the extent where motor fusion is achieved. Furthermore, with regard to the measurement subject who was found to have strong motor fusion based on the results of measurement, the Panum's fusional area may be wide, and thus it is possible to provide an eyeglass lens provided with a lens design that can reduce inset without affecting fusion of images and reduce the maximum aberration on the nasal side, for example. Note that "motor fusion" refers to fusion with eyeball movements performed to maintain single vision.

<Example of system configuration>

Next, a specific content of the present embodiment will be described.

(Configuration of eyeglass lens manufacturing system)

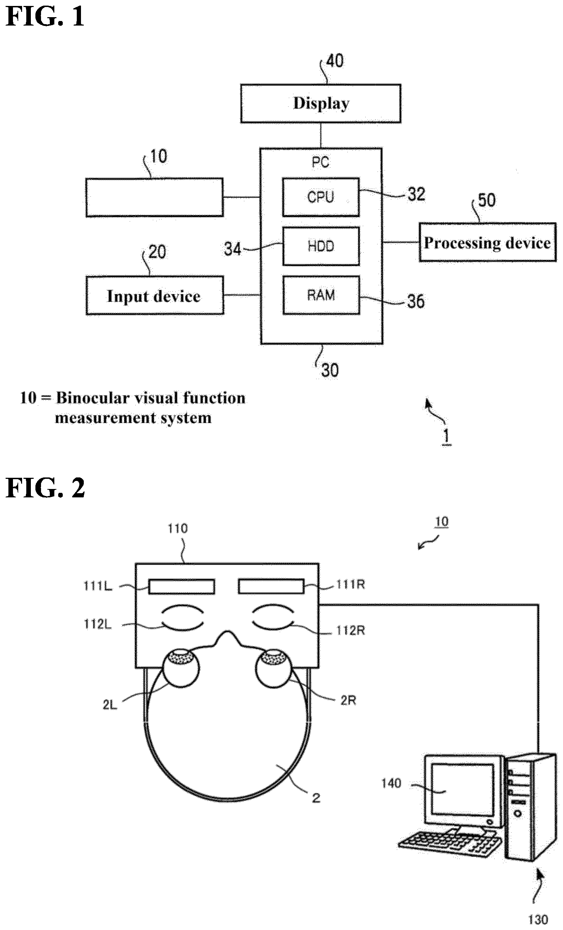

FIG. 1 is a block diagram showing a configuration of an eyeglass lens manufacturing system 1 for realizing an eyeglass lens manufacturing method according to the present embodiment. The eyeglass lens manufacturing system 1 is installed in an eyeglass lens manufacturing factory, for example, and as shown in FIG. 1, the eyeglass lens manufacturing system 1 includes a binocular visual function measurement system 10, an input device 20 (a keyboard, a mouse, a game controller, and the like), a PC (Personal Computer) 30, a display 40, and a processing device 50. The PC 30 receives data regarding measurement of the binocular visual function of a measurement subject measured using the binocular visual function measurement system 10, and data regarding the specifications of an eyeglass lens that was input to the input device 20. Specification data includes the optical properties and product type of eyeglass lens, for example. Vertex refractive power (spherical refractive power, cylindrical power, cylindrical axis direction, prismatic refractive power, and prism base direction) and the like are considered as being the optical properties of an eyeglass lens, for example. The binocular visual function measurement system 10 and the input device 20 may be installed in an optician's store away from an eyeglass lens manufacturing plant. In this case, data measured by the binocular visual function measurement system 10 and the specification data input to the input device 20 are transmitted to the PC 30 via a computer network.

The PC 30 includes a CPU (Central Processing Unit) 32, an HDD (Hard Disk Drive) 34, and a RAM (Random Access Memory) 36. A processing control program for controlling the processing device 50 is installed in the HDD 34. The CPU 32 loads the processing control program onto the RAM 36, and starts the program. When the processing control program is started, a GUI (Graphical User Interface) for issuing an instruction to design and manufacture an eyeglass lens is displayed on a display screen of the display 40. The processing control program selects a semi-finished lens based on specification data and measurement data, performs surface shape optimization calculation, and determines optical design values.

An operator sets the selected semi-finished lens in the processing device 50, operates the GUI, and inputs an instruction to start processing. The processing control program reads the determined optical design values and controls driving of the processing device 50. The processing device 50 grinds the surface of the semi-finished lens according to the execution of the processing control program so as to manufacture an eyeglass lens. Note that a specific method for designing an eyeglass lens using measurement data regarding the binocular visual function is described in a pamphlet in WO 2010/090144 filed by the applicant, for example.

(Configuration of binocular visual function measurement system)

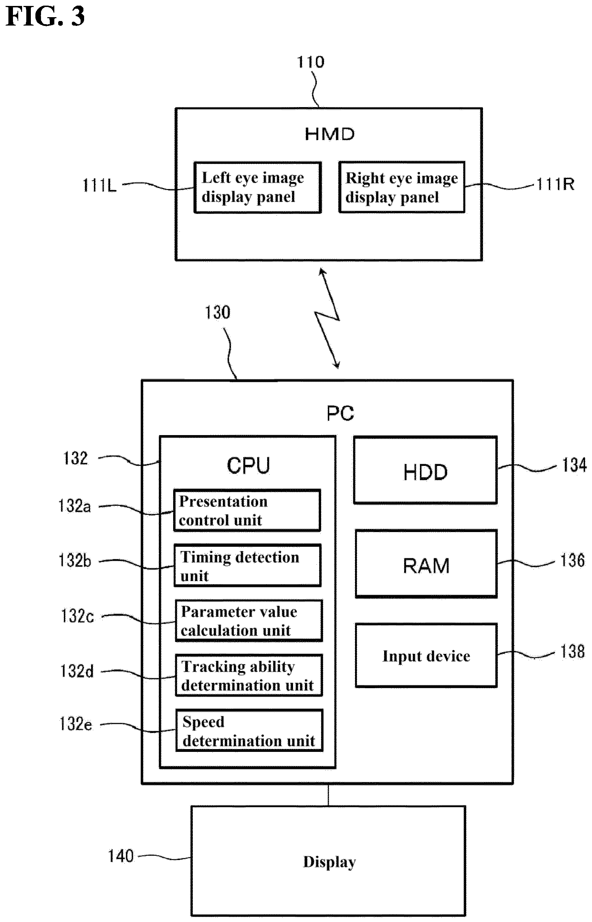

FIG. 2 is a diagram showing an overview of the binocular visual function measurement system 10. FIG. 3 is a block diagram showing a configuration of the binocular visual function measurement system 10. In the binocular visual function measurement method using the binocular visual function measurement system 10, multiple types of binocular visual functions of a measurement subject 2 can be measured in order to obtain design data (or evaluation data) regarding an eyeglass lens that cannot be obtained using a prescription obtained focusing on only one eye.

As shown in FIGS. 2 and 3, the binocular visual function measurement system 10 includes an HMD 110. The HMD 110 has a right eye image display panel 111R and a left eye image display panel 111L in a housing that is worn on the head of the measurement subject 2. Then, a configuration is adopted in which the right eye image display panel 111R displays the right eye image to be viewed by the right eye 2R of the measurement subject 2, and the left eye image display panel 111L displays the left eye image to be viewed by the left eye 2L of the measurement subject 2 in the state where the HMD 110 is worn by the measurement subject 2. In the HMD 110 having such a configuration, the right eye image display panel 111R and the left eye image display panel 111L are disposed in the closed space formed by the housing, and thus, images are displayed to the measurement subject 2 in a space where information (real space information) that gives a sense of depth and perspective is blocked out from the outside world.

That is, the HMD 110 functions as a "visual target presentation unit" to present the right eye image and the left eye image to the measurement subject 2 in a space where real space information is blocked out. In other words, the visual target presentation unit is constituted using the HMD 110 to be worn by the measurement subject 2.

When such an HMD 110 is worn, only the right eye image is visible to the right eye 2R of the measurement subject 2, and only the left eye image is visible to the left eye 2L of the measurement subject 2. As a result, the measurement subject 2 can three-dimensionally perceive the parallax images on the right eye image display panel 111R and the left eye image display panel 111L, the parallax images being formed at non-corresponding points on the retina within the Panum's fusional area.

Note that the HMD 110 may have the function of being able to vary the settings of the visual range of the measurement subject 2 with respect to the right eye image and the left eye image. Specifically, in order to achieve a variable visual range, the HMD 110 may have display optical systems 112R and 112L that include eyepieces and the like, for example. Furthermore, a configuration may be adopted in which a plurality of spacers (frame members) are prepared, and the visual range of the HMD 110 can be varied by selectively installing any of the plurality of spacers, for example.

A PC 130 is connected to the HMD 110 configured as described above via a wired or wireless communication line. A display 140 is connected to the PC 130.

The PC 130 includes a CPU 132, an HDD 134, a RAM 136, and an input device 138 (a keyboard, a mouse, a game controller, and the like). A binocular visual function measurement program for measuring a binocular visual function is installed in the HDD 134. The CPU 132 loads the binocular visual function measurement program onto the RAM 136, and starts the program. When the binocular visual function measurement program is started, the CPU 132 functions as a presentation control unit 132a, a timing detection unit 132b, and a parameter value calculation unit 132c.

The presentation control unit 132a controls image display operations of the right eye image display panel 111R and the left eye image display panel 111L of the HMD 110. Specifically, the presentation control unit 132a instructs the right eye image display panel 111R to present a right eye image and instructs the left eye image display panel 111L to present a left eye image, and the presentation control unit 132a changes positions where the right eye image and the left eye image are presented, relative to each other. A specific mode in which the presentation positions are changed relative to each other will be described later in detail.

The right eye image and the left eye image presented by the presentation control unit 132a function as parallax images, and thus it is presumed that they are formed using figures having the same shape and the same size. Note that specific examples of the figures constituting parallax images will be described later in detail.

When the presentation positions of the right eye image and the left eye image are changed relative to each other, the timing detection unit 132b detects the timing at which the measurement subject 2 cannot fuse the right eye image and the left eye image. Such timing may be detected based on the content of an operation made by the measurement subject 2 on the input device 138.

When the parameter value calculation unit 132c detects the timing at which the timing detection unit 132b detects that images cannot be fused, the parameter value calculation unit 132c calculates a predetermined parameter value for the binocular visual function of the measurement subject 2 based on the relationship between the relative positions of the right eye image and the left eye image. Specific examples of the predetermined parameter value will be described later in detail.

In addition to these functions, the CPU 132 may function as a tracking ability determination unit 132d and a speed determination unit 132e in response to the start of the binocular visual function measurement program.

The tracking ability determination unit 132d determines the level of the tracking ability of an eye of the measurement subject 2 with respect to a change in the position of a presented image. Specifically, the tracking ability determination unit 132d presents a visual target that successively moves to the right eye image display panel 111R or the left eye image display panel 111L before measuring the binocular visual function of the measurement subject 2, for example. Then, based on the content of an operation made by the measurement subject 2 who has seen the visual target on the input device 138, it is determined which level the tracking ability of the eye of the measurement subject 2 corresponds to out of multiple preset levels. However, the determination method is not limited to such a method, and the tracking ability determination unit 132d may be configured to determine the tracking ability of an eye using another method. Because HMD devices having a line-of-sight tracking function are available as products, for example, when the HMD 110 has a line-of-sight tracking function, a configuration may be adopted in which the level of the trackability of the left and right eyes of the measurement subject 2 is determined by tracking the line-of-sight of the eyes of the measurement subject 2 using the line-of-sight tracking function.

The speed determination unit 132e determines the speed of a change in the relative positions of the right eye image and the left eye image when measuring the binocular visual function of the measurement subject 2, based on the results of the determination made by the tracking ability determination unit 132d. Specifically, when the tracking ability of the eyes of the measurement subject 2 is of a high level, the speed determination unit 132e determines the speed of a change in the relative positions of the right eye image and the left eye image as a high speed, whereas, when the tracking ability of the eyes of the measurement subject 2 is of a low level, the speed determination unit 132e determines the speed of a change in the relative positions of the right eye image and the left eye image as a low speed. The speed determination unit 132e instructs the presentation control unit 132a to control image display operations according to the results of the determination therefor.

Note that, in the system configuration described above, if all of the constituent elements of the eyeglass lens manufacturing system 1 are installed at the same location, the PC 30 shown in FIG. 1 and the PC 130 shown in FIG. 2 or 3 may be a single PC. Also, the input device 20 shown in FIG. 1 and the input device 138 shown in FIG. 2 or 3 may be a single input device. Furthermore, the display 40 shown in FIG. 1 and the display 140 shown in FIG. 2 or 3 may be a single display.

<Procedure for measuring binocular visual function>

Next, specific content of a procedure for measuring a binocular visual function using the binocular visual function measurement system 10 configured as described above, that is, the binocular visual function measurement method according to the present embodiment, will be described.

If a binocular visual function is to be measured, the binocular visual function measurement program is started in the PC 130, and the GUI for giving various instructions for measuring the binocular visual function is displayed on the display screen of the display 140. Also, when the operator operates the GUI, the binocular visual function measurement program generates measurement data in accordance with the GUI operation and outputs the data to the HMD 110. The HMD 110 processes measurement data received from the PC 130, generates the right eye image and the left eye image for measuring the binocular visual function, and displays the images on the right eye image display panel 111R or the left eye image display panel 111L. This starts the measurement of the binocular visual function.

The binocular visual function measurement program supports various measurement items relating to the binocular visual function, and outputs the parameter values for the various measurement items as the results of the measurement. Examples of the supported parameter values include the convergence range, the left-right eye vertical divergence allowable value, the first unequal magnification allowable value, the second unequal magnification allowable value, and the left-right eye rotation parallax allowable value. When the operator measures the binocular visual function, the operator selects any one of the measurement items on the GUI.

Furthermore, the operator inputs the age, visual range, and the like as measurement conditions. The input measurement conditions are stored in the HDD 134. Note that, with regard to the visual range, the visual range may be changed using an eyepiece or the like as needed if the HMD 110 has the function of being able to vary the visual range settings.

The following describes processing executed by the binocular visual function measurement program when each of the above-listed measurement items is selected.

(If "convergence range" is selected)

The binocular visual function measurement program transitions to the convergence range measurement mode in which the convergence range of the measurement subject 2 is measured. The "convergence range" herein refers to convergence without accommodation. Here, as indicated by a known Donders diagram, the convergence (or divergence) of eyeballs and accommodation originally occur together. Therefore, it is not easy to measure convergence separately from accommodation. Note that the Donders diagram is described in a document "written by Shinobu Ishihara and revised by Shinichi Shikano, "Little Pupil Science" 17th revised version, Kanehara & Co. Ltd., (1925) p50", a document "written by Toyohiko Hatada, "Depth Information and Characteristics of Vision", Visual Information Research Group, April 23, 1974, p12", and WO 2010/090144 pamphlet filed by the applicant, and the like. In the Donders diagram, a straight line passing through the origin and having a slope 1 (angle of 45 degrees) is the Donders line. The Donders line represents cooperation between convergence and accommodation when a measurement subject who does not have strabismus or heterophoria is looking at an object with naked eyes. A Donders curve indicating the limit of the convergence (or the divergence) is plotted on the left and right sides of the Donders line. Values from one point on the Donders line to the right Donders curve (the side where the convergence angle is large) are classified into negative relative convergence, and values from one point thereon to the left Donders curve (the side where the convergence angle is small) are classified into positive relative convergence.

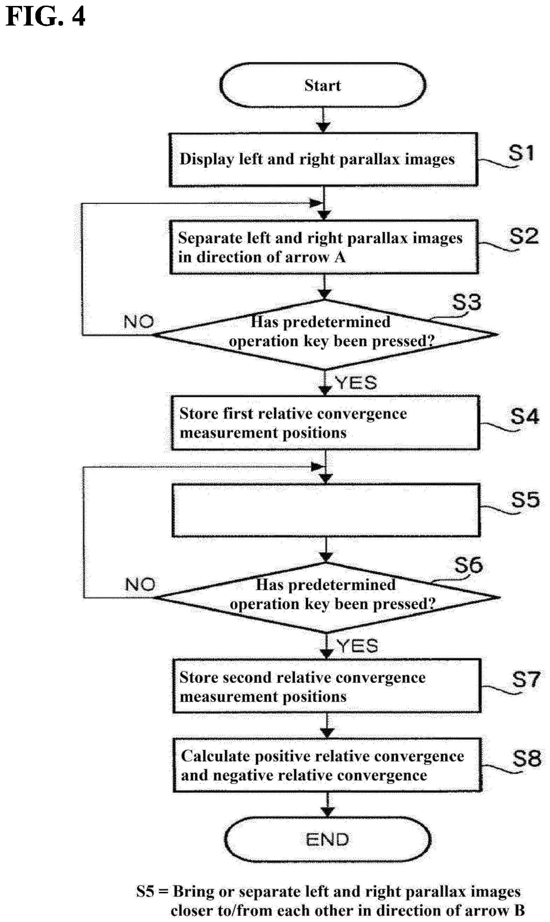

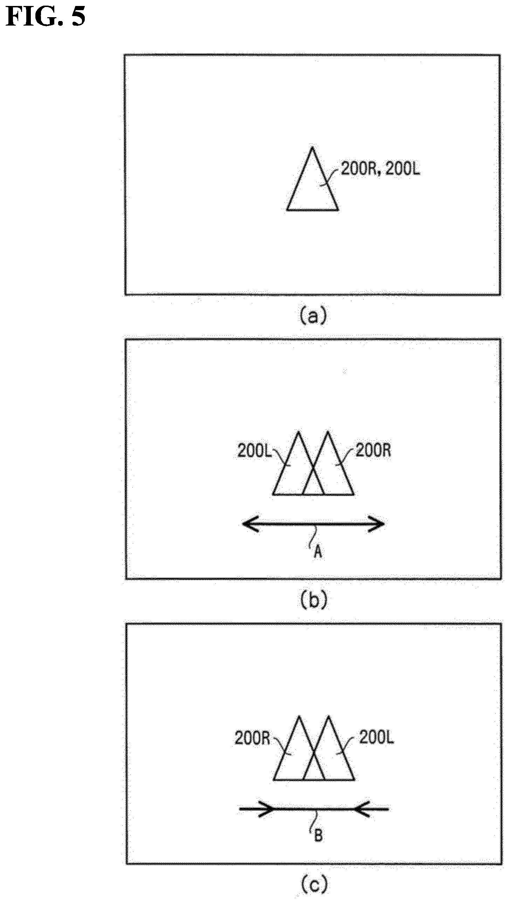

FIG. 4 is a diagram showing a flowchart of processing executed by the binocular visual function measurement program in a convergence range measurement mode. FIG. 5 shows transition diagrams of images displayed on the display screen during execution of the convergence range measurement mode. A processing step is abbreviated as "S" in the following description of this specification and drawings.

As shown in FIG. 5(a), when transitioning to the convergence range measurement mode, the left eye image 200L and the right eye image 200R are displayed on the left eye image display panel 111L and the right eye image display panel 111R such that these images are visible in an overlapping state (S1 in FIG. 4). The left eye image 200L and the right eye image 200R are identical images with the same size, color, shape, and the like. It is desirable that the left eye image 200L and the right eye image 200R have a simple geometrical shape such that the measurement subject 2 can focus on measurement. Although the drawings show a case where the left eye image 200L and the right eye image 200R are triangular figures, there is no limitation to this, and these images may be figures of a straight line (line segment) extending in the same direction over the same length, figures with other geometrical shapes, or the like, for example.

Furthermore, the measurement subject 2 is instructed to press a predetermined operation key of the input device 138 when the measurement subject 2 sees two images. An instruction is displayed on at least one of the right eye image display panel 111R and the left eye image display panel 111L, for example. Also, an operator may give an instruction directly to the measurement subject 2. The same instruction is issued even when measurement items other than the convergence range are measured.

As shown in FIG. 5(b), in the processing of S2 in FIG. 4, the left eye image 200L and the right eye image 200R move in the horizontal direction (the direction indicated by the arrow A in FIG. 5(b)) of the right eye image display panel 111R and the left eye image display panel 111L, and separate from each other. The movements of images that separate from each other are rendered in a continuously changing manner or in an incrementally changing manner. The left eye image 200L and the right eye image 200R continue to separate from each other until the predetermined operation key of the input device 138 is pressed (S2, NO in S3 in FIG. 4). When the predetermined operation key is pressed by the measurement subject 2 (YES in S3 in FIG. 4), the amounts of positional shift of the left eye image 200L and the right eye image 200R at this time (hereinafter referred to as "first relative convergence measurement positions" for convenience of description) are stored in the HDD 134 (S4 in FIG. 4). Note that the positions of the left eye image 200L and the right eye image 200R need only relatively separate from each other. Therefore, the position of either the left eye image 200L or the right eye image 200R may be fixed during measurement. The same applies to a left-right eye vertical divergence allowable value measurement mode, which will be described later.

The period of time during which separating images are presented is, for example, about one second at the most, with the burden on the measurement subject 2 taken into consideration. Note that the presentation period of time can be changed by the operator as needed.

In the processing of S5 in FIG. 4, the left eye image 200L and the right eye image 200R move from the first relative convergence measurement positions in the horizontal direction (the direction indicated by the arrow B in FIG. 5(c) that is the opposite of the direction indicated by the arrow A) of the right eye image display panel 111R and the left eye image display panel 111L, and approach each other. The left eye image 200L and the right eye image 200R approach each other and temporarily overlap each other, and as shown in FIG. 5(c), continue to move in the direction indicated by the arrow B and then separate from each other. The left eye image 200L and the right eye image 200R continue to approach each other or separate from each other until the predetermined operation key of the input device 138 is pressed (S5, NO in S6 in FIG. 4). When the predetermined operation key is pressed by the measurement subject 2 (YES in S6 in FIG. 4), the amounts of positional shift of the left eye image 200L and the right eye image 200R at this time (hereinafter referred to as "second relative convergence measurement positions" for convenience of description) are stored in the HDD 134 (S7 in FIG. 4).

In the processing of S8 in FIG. 4, a first convergence angle (divergence limit) is calculated using the first relative convergence measurement positions and the visual range. A second convergence angle (convergence limit) is calculated using the second relative convergence measurement positions and the visual range. The first convergence angle indicates the positive relative convergence corresponding to accommodation at the visual range during measurement. The second convergence angle indicates the negative relative convergence corresponding to accommodation at the visual range during measurement. The visual range does not change during measurement. Therefore, the accommodation of the measurement subject 2 does not change substantially during measurement. Thus, the positive relative convergence and the negative relative convergence can be very easily measured with high accuracy while separating the positive relative convergence and the negative relative convergence from the accommodation.

When the positive relative convergence and the negative relative convergence that are obtained in the processing of S8 in FIG. 4 are applied to the Donders diagram, the left and right Donders curves are predicted. That is, the relationship regarding cooperation between the convergence and the accommodation of the measurement subject 2 can be obtained. A Donders curve changes depending on age. Therefore, when a Donders curve is predicted, it is preferable to consider the age input as a measurement condition.

When measurement is performed in the convergence range measurement mode while changing the visual range, the positive relative convergence and the negative relative convergence obtained when different accommodation occurs are measured. As the measurement of the convergence range at different visual ranges is repeated, the number of pieces of collected sample data for predicting the Donders curves increases. Therefore, the relationship regarding cooperation between the convergence and the accommodation of measurement subject 2 can be obtained more accurately.

If the separation speed between the left eye image 200L and the right eye image 200R is low, it is presumed that the measurement subject 2 will move the muscles around his/her eyeballs more strongly than in daily life to forcibly fuse the images. At this time, the fusional area expands beyond the range assumed considering the natural eyeball movement, and thus measurement accuracy decreases. In view of this, the separation speed is set relatively high such that the measurement subject 2 can see the left eye image 200L and the right eye image 200R in a relaxed state, which is similar to that in daily life. From a similar viewpoint, the speed of the relative changes (movement, rotation, scaling, and the like) between the left eye image 200L and the right eye image 200R is set relatively high when measuring measurement items other than the convergence range.

The operator can set and change the speed of relative changes as appropriate. However, it is desirable that the speed of a relative change that can be set and changed is within a predetermined range of speed. The upper limit of the speed of a relative change is set to a value such that an error caused by a time lag between the timing at which the measurement subject cannot fuse images and the timing at which the predetermined operation key of the input device 138 is pressed is within a predetermined allowable value range. On the other hand, the lower limit may be set to a value such that the display of the images changes before the fusional area expands beyond the range assumed considering the natural eyeball movement due to fusion of images being facilitated, for example. Specific examples of the set upper and lower limits are determined after performing experiments and the like, for example.

Note that, if the binocular visual function measurement program realizes the function of the tracking ability determination unit 132d and the speed determination unit 132e, the level of the tracking ability of the eyes of the measurement subject 2 with respect to a change in the positions of the presented images may be determined and the speed of a change in the relative positions of the left eye image 200L and the right eye image 200R may be determined based on the results of the determination. Doing this makes it possible to reflect the level of the tracking ability of the eyes of the measurement subject 2 on the moving speeds of the left eye image 200L and the right eye image 200R, and thus the measurement subject 2 can move his/her eyeballs without difficulty, and as a result, it is possible to accurately measure the binocular visual function of the measurement subject 2.

The left eye image 200L and the right eye image 200R may be separated from each other multiple times in order to measure the convergence range quickly and accurately. At the first measurement (hereinafter referred to as "pre-measurement" for convenience of description), for example, the left eye image 200L and the right eye image 200R are separated from each other at a high speed so as to specify the approximate position of the fusional limit. In the second measurement (hereinafter referred to as "main measurement" for convenience of description), the left eye image 200L and the right eye image 200R are separated from each other at a low speed (note that, a speed at which fusion is not strong) near the approximate position specified in the pre-measurement. In the main measurement, the separation speed is low, and thus an error caused by a time lag between the timing at which the measurement subject 2 cannot fuse images and the timing at which the predetermined operation key of the input device 138 is pressed is suppressed, and measurement accuracy is improved. Furthermore, the measurement interval in the main measurement is limited to the vicinity of the approximate position of the fusional limit specified in the pre-measurement. Thus, the convergence range can be measured quickly even if pre-measurement and main measurement are performed. Measurement items other than the convergence range are quickly measured with high accuracy, and thus pre-measurement and main measurement may be performed.

The parameter values of the convergence range of the measurement subject 2 are obtained from the positive relative convergence and the negative relative convergence that are measured in the convergence range measurement mode. The potential shift (esotropia or exotropia) of the measurement subject 2 is estimated based on such parameter values, for example. Parameter values can be estimated for measurement items other than the convergence range in a similar manner.

(If "left-right eye vertical divergence allowable value" is selected)

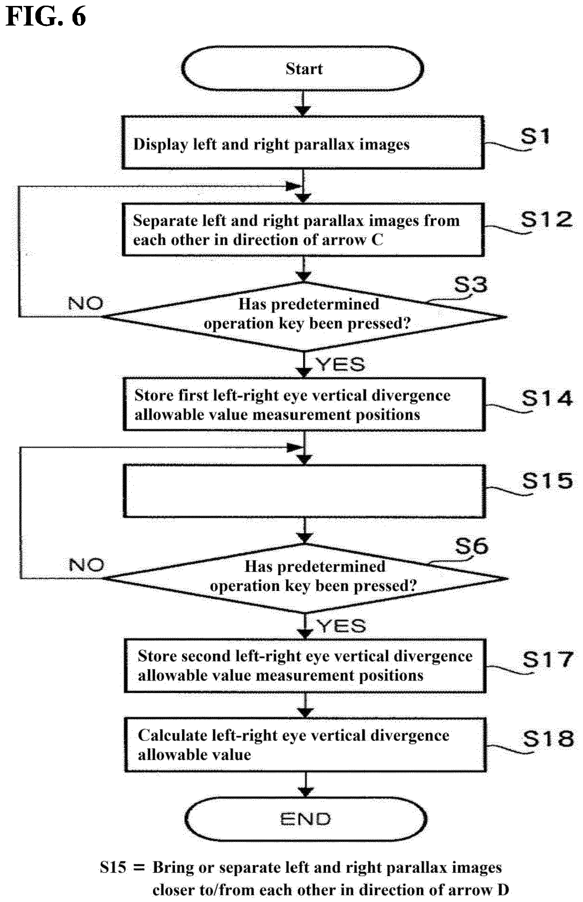

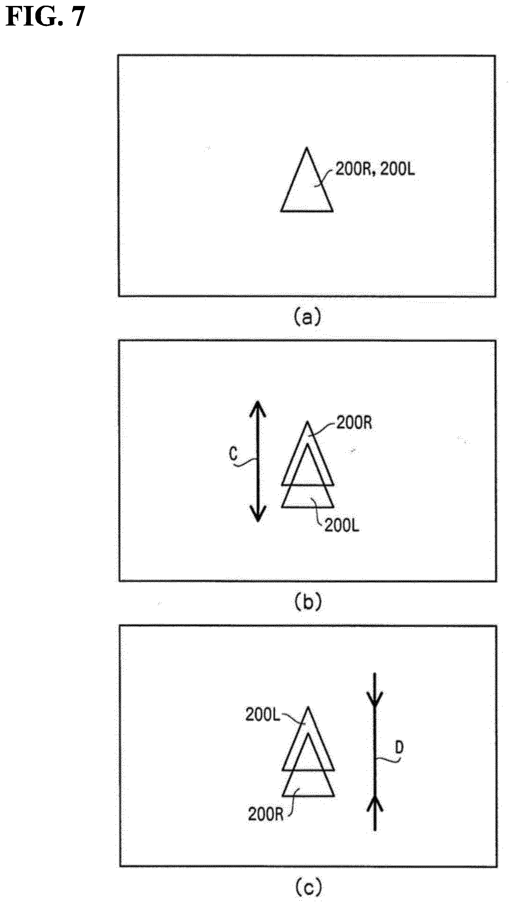

The binocular visual function measurement program transitions to the left-right eye vertical divergence allowable value measurement mode in which the left-right eye vertical divergence allowable value of the measurement subject 2 is measured. The left-right eye vertical divergence allowable value is the allowable value of vertical divergence of the left and right eyes that can realize/enable stereoscopic vision. FIG. 6 is a diagram showing a flowchart of processing executed by the binocular visual function measurement program in the left-right eye vertical divergence allowable value measurement mode. FIG. 7 shows transition diagrams of images displayed on the display screen during execution of the left-right eye vertical divergence allowable value measurement mode. In the following description of this specification and the drawings, the same or similar processes are given the same or similar reference numerals, and description thereof will be simplified or omitted.

When the binocular visual function measurement program transitions to the left-right eye vertical divergence allowable value measurement mode and images are displayed (S1 in FIG. 6, FIG. 7(a)), as shown in FIG. 7(b), the left eye image 200L and the right eye image 200R move in the vertical direction on the display screen (the direction indicated by the arrow C in FIG. 7(b)) and separate from each other (S12 in FIG. 6). When the predetermined operation key is pressed by the measurement subject 2 (YES in S3 in FIG. 6), the amounts of positional shift of the left eye image 200L and the right eye image 200R at this time (hereinafter referred to as "first left-right eye vertical divergence allowable value measurement positions" for convenience of description) are stored in the HDD 134 (S14 in FIG. 6).

In the processing of S15 in FIG. 6, the left eye image 200L and the right eye image 200R move in the vertical direction on the display screen (the direction indicated by the arrow D shown in FIG. 7(c) that is the opposite of the direction indicated by the arrow C) from the first left-right eye vertical divergence allowable value measurement positions and approach each other, and separate from each other as shown in FIG. 7(c). When the predetermined operation key is pressed by the measurement subject 2 (YES in S6 in FIG. 6), the amounts of positional shift of the left eye image 200L and the right eye image 200R at this time (hereinafter referred to as "second left-right eye vertical divergence allowable value measurement positions" for convenience of description) are stored in the HDD 134 (S17 in FIG. 6).

In the processing of S18 in FIG. 6, the left-right eye vertical divergence allowable value and a vertical range in which images of an object can be fused in the visual range is calculated based on the first and second left-right eye vertical divergence allowable measurement positions and the visual range. When measurement is performed in the left-right eye vertical divergence allowable value measurement mode while changing the visual range, the left-right eye vertical divergence allowable value obtained when different accommodation occurs (e.g., when a subject looks at a near position or distant position) is measured.

(If "first unequal magnification allowable value" is selected)

The first unequal magnification allowable value is the allowable value of unequal magnification of the left and right eyes that can enable stereoscopic vision. In general, whether or not to prepare a prescription of eyeglass lenses with respect to the unequal magnification is determined in a pattern-like manner in accordance with whether the eyesight difference between the left and right eyes is larger than or equal to 2 diopters. However, there are individual differences between patients, and thus, it may be difficult for a patient to achieve fusion of images even if the eyesight difference between the left and right eyes is less than 2 diopters. Also, in contrast to this, even if the eyesight difference between the left and right eyes is larger than or equal to 2 diopters, there are also cases where it may not be difficult for a patient to achieve fusion of images. In the first unequal magnification allowable value measurement mode described later, whether or not it is possible to fuse images is measured considering the eyesight difference between the left and right eyes. Thus, when the results of measurement obtained in the first unequal magnification allowable value measurement mode are used, it is possible to prepare a prescription optimal for unequal magnification with individual differences taken into consideration.

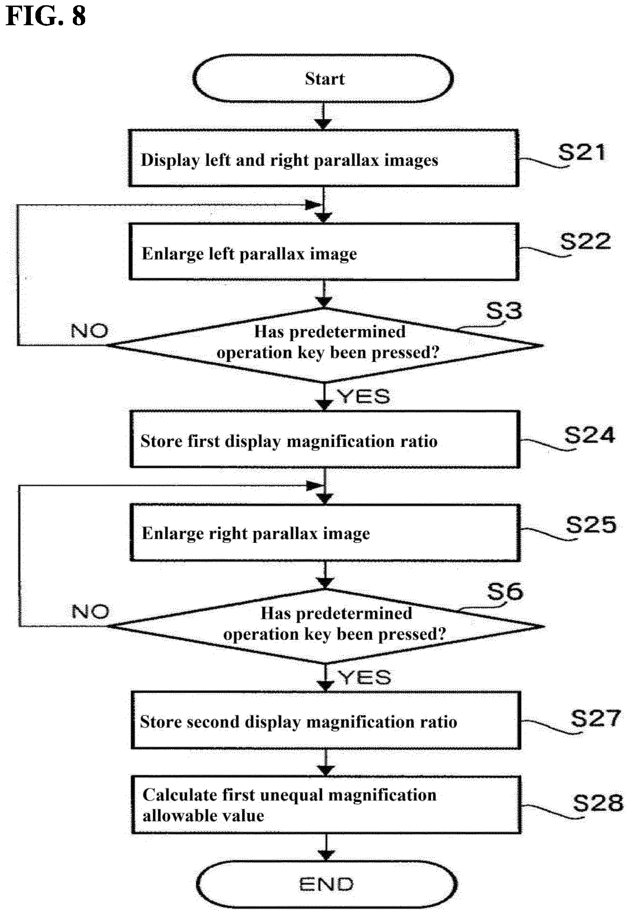

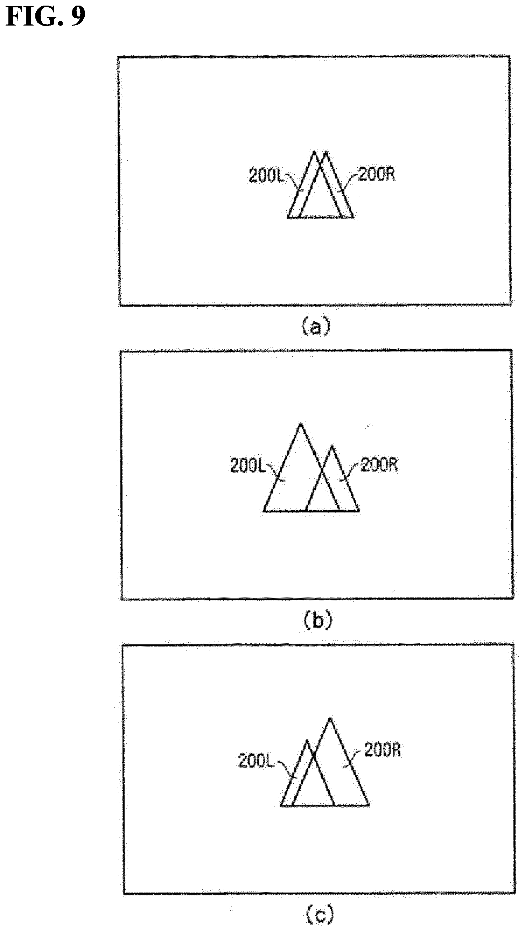

The binocular visual function measurement program transitions to the first unequal magnification allowable value measurement mode in which the first unequal magnification allowable value of the measurement subject 2 is measured. FIG. 8 is a diagram showing a flowchart of processing executed by the binocular visual function measurement program in the first unequal magnification allowable value measurement mode. FIG. 9 shows transition diagrams of images displayed on the display screen during execution of the first unequal magnification allowable value measurement mode.

When the binocular visual function measurement program transitions to the first unequal magnification allowable value measurement mode, as shown in FIG. 9(a), the left eye image 200L and the right eye image 200R are displayed at slightly shifted positions in the fusional area of the measurement subject 2 (S21 in FIG. 8). The positions where the left eye image 200L and the right eye image 200R are displayed are determined with reference to the results of measurement of the convergence range and the left-right eye vertical divergence allowable value. If the convergence range and the left-right eye vertical divergence allowable value have not been measured, the operator performs minute adjustment such that the positions of the left eye image 200L and the right eye image 200R are located in the fusional area of the measurement subject 2.

As shown in FIG. 9(b), in the processing of S22 in FIG. 8, the left eye image 200L is enlarged with respect to the right eye image 200R. The enlargement ratio of the left eye image 200L is fixed with regard to the aspect ratio, and the left eye image 200L is rendered in a continuously changing manner or an incrementally changing manner. The displayed left eye image 200L continues to be enlarged until the predetermined operation key of the input device 138 is pressed (S22 and NO in S3 in FIG. 8). When the predetermined operation key is pressed by the measurement subject 2 (YES in S3 in FIG. 8), the magnification ratio between the left eye image 200L and the right eye image 200R displayed at this time (hereinafter referred to as "first display magnification ratio" for convenience of description) is stored in the HDD 134 (S24 in FIG. 8). Note that, in the first unequal magnification allowable value measurement mode, it is sufficient that the display magnification ratio between the left eye image 200L and the right eye image 200R changes relative to each other. Therefore, a change made to images may be reduction, instead of enlargement. Furthermore, during measurement, the left eye image 200L and the right eye image 200R may be enlarged or reduced simultaneously at different scaling rates. The same applies to the second unequal magnification allowable value measurement mode, which will be described later.

As shown in FIG. 9(c), in the processing of S25 in FIG. 8, the right eye image 200R is enlarged with respect to the left eye image 200L. When the predetermined operation key is pressed by the measurement subject 2 (YES in S6 in FIG. 8), the magnification ratio between the left eye image 200L and the right eye image 200R displayed at this time (hereinafter referred to as "second display magnification ratio" for convenience of description) is stored in the HDD 134 (S27 in FIG. 8).

In the processing of S28 in FIG. 8, based on the first and second display magnification ratios and the visual range, the first unequal magnification allowable value in the visual range is calculated. When measurement is performed in the first unequal magnification allowable value measurement mode while changing the visual range, the first unequal magnification allowable value obtained when different accommodation occurs (e.g., when a subject looks at a near position or distant position) is measured.

(If "second unequal magnification allowable value" is selected)

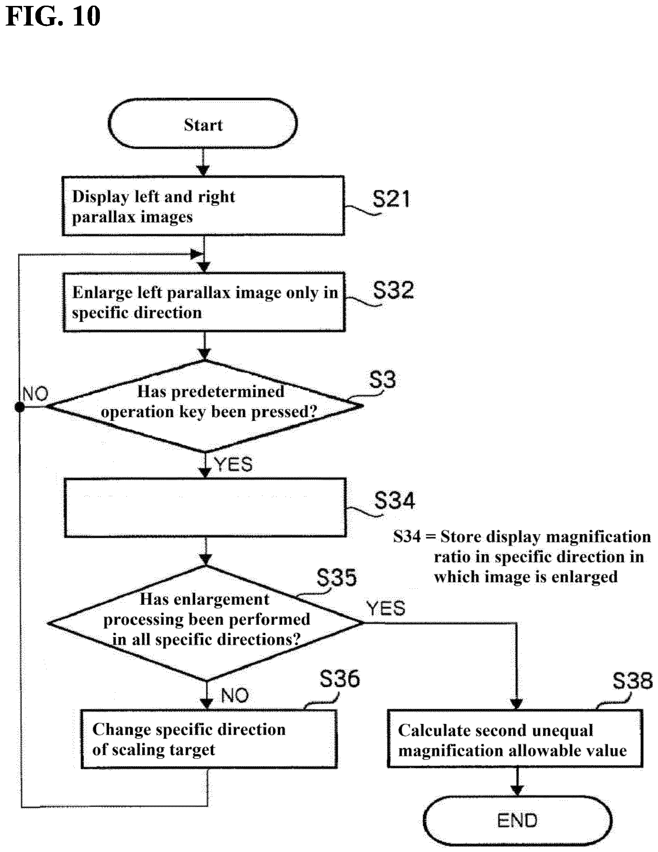

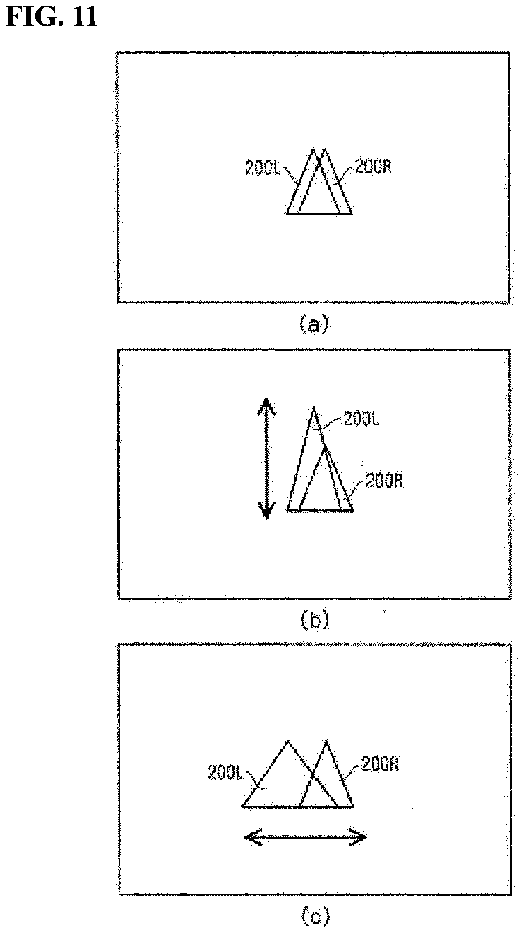

The binocular visual function measurement program transitions to the second unequal magnification allowable value measurement mode in which the second unequal magnification allowable value of the measurement subject 2 is measured. The second unequal magnification allowable value is the allowable value of unequal magnification of the left and right eyes that can enable stereoscopic vision limited to a specific direction. FIG. 10 is a diagram showing a flowchart of processing executed by the binocular visual function measurement program in the second unequal magnification allowable value measurement mode. FIG. 11 shows transition diagrams of images displayed on the display screen during execution of the second unequal magnification allowable value measurement mode.

When the binocular visual function measurement program transitions to the second unequal magnification allowable value measurement mode and images are displayed (S21 in FIG. 10, FIG. 11(a)), the left eye image 200L displayed in a specific direction is enlarged with respect to the right eye image 200R (S32 in FIG. 10). In the image example shown in FIG. 11(b), the display magnification of the left eye image 200L is increased only in the vertical direction on the screen. The enlargement of the left eye image 200L is rendered such that the size thereof changes continuously or incrementally. The displayed left eye image 200L continues to be enlarged until the predetermined operation key of the input device 138 is pressed (S32 and NO in S3 in FIG. 10). When the predetermined operation key is pressed by the measurement subject 2 (YES in S3 in FIG. 10), the display magnification ratio between the left eye image 200L and the right eye image 200R in the vertical direction on the screen at this time (hereinafter referred to as "first specific direction display magnification ratio" for convenience of description) is stored in the HDD 134 (S34 in FIG. 10).

In the processing of S35 in FIG. 10, it is determined whether or not the left eye image 200L has been enlarged in all of the specific directions of scaling targets. In the present embodiment, the directions of the scaling targets are two directions including the vertical direction on the screen and the horizontal direction on the screen. Therefore, the direction of the scaling target is changed to the horizontal direction on the screen (NO in S35 and S36 in FIG. 10). Then, as shown in FIG. 11(c), the display of the left eye image 200L in the horizontal direction on the screen is enlarged with respect to the right eye image 200R. When the predetermined operation key is pressed by the measurement subject 2 (YES in S3 in FIG. 10), the display magnification ratio between the left eye image 200L and the right eye image 200R in the horizontal direction on the screen at this time (hereinafter referred to as "second specific direction display magnification ratio" for convenience of description) is stored in the HDD 134 (S34 in FIG. 10).

In the processing of S38 in FIG. 10, the second unequal magnification allowable value in the visual range is calculated based on the first and second specific direction display magnification ratios and the visual range. When measurement is performed in the second unequal magnification allowable value measurement mode while changing the visual range, the second unequal magnification allowable value obtained when different accommodation occurs (e.g., when a subject looks at a near position or distant position) is measured. The directions of the scaling targets are not limited to two directions including the horizontal direction on the screen or the vertical direction on the screen, and may include other directions.

(If "left-right eye rotation parallax allowable value" is selected)

Fusional rotation may occur when the line-of-sight directions such as convergence are not parallel with each other. The rotation of an eyeball in the distance vision is based on Listing's law. Listing's law is a law defining the posture of an eyeball when the eyeball faces in a given direction in a space. The posture of the eyeball indicates the orientation of the eyeball in the lateral direction and the longitudinal direction. If the posture of the eyeball is not defined, upward, downward, left, and right directions of a retinal image are not defined. The posture of the eyeball is not defined uniquely by only the line-of-sight direction, that is, the direction of an optical axis of the eyeball. The posture of the eyeball can take all of the directions defined in regard to rotation about the line of sight serving as an axis even when the line-of-sight direction is defined.

Listing's law defines the posture of an eyeball facing an infinitely distant point in a given line-of-sight direction. With regard to Listing's law, "it is conceivable that any rotation of a single eye may occur about an axis in one plane (Listing's plane)" is described in "Handbook of Visual Information Processing" p. 405, for example.

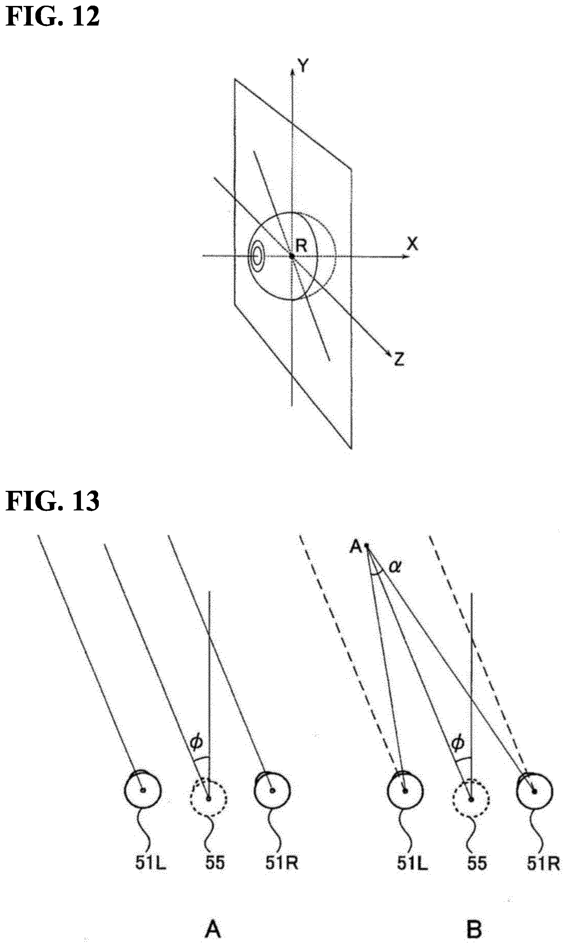

The aforementioned Listing's law will be described using a coordinate system shown in FIG. 12. The coordinate system shown in FIG. 12 is a coordinate system where a point R, which is the rotation center of an eyeball, is the origin, and a direction extending from the front plane (horizontal front side) and entering the eye is defined as the X-axial direction, a vertical direction orthogonal to the X-axial direction is defined as the Y-axial direction, and a horizontal direction orthogonal to the X-axial direction is defined as the Z-axial direction. The Y-Z plane is the Listing's plane.

The posture after rotation of an eyeball in a given direction is the same as rotation about a straight line serving as an axis in the Listing's plane including the point R. In FIG. 12, an example of straight lines serving as rotation axes are illustrated between the Y-axis and the Z-axis (on the Y-Z plane). The rotation axes are orthogonal to each of the primary eye position (X-axial direction) and the line-of-sight direction after rotation. Here, a case where an eyeball is rotated to direction vectors (L, M, N) that are not shown is considered. In this case, the vectors in the X-axial direction, the Y-axial direction, and the Z-axial direction in the eyeball coordinate system after rotation are calculated using the following equation (1).

[Mathematical 1]

$$x = L\,i + M\,j + N\,k$$

$$y = -M\,i + \left(1 - \frac{M^2}{1+L}\right)j - \frac{MN}{1+L}\,k$$

$$z = -N\,i - \frac{MN}{1+L}\,j + \left(1 - \frac{N^2}{1+L}\right)k \qquad \cdots (1)$$

Listing's law is appropriate in regard to a case where a single eye defines the posture of an eyeball with respect to an object at an infinite distance. Also, if a subject leans his/her body while looking at an object at an infinite distance, for example, the eyeballs of the left eye and the right eye have the same posture and the same rotation. In contrast, if a subject looks at an object that is not at an infinite distance with his/her eyes, the eyeballs of the left eye and the right eye may have different postures.

FIGS. 13A and 13B are diagrams illustrating the line-of-sight direction of the left and right eyes in the case of binocular vision. In each of FIGS. 13A and 13B, a dashed line represents a virtual eyeball 55 arranged at an intermediate position between a left eyeball 51L and a right eyeball 51R. As shown in FIG. 13A, if a subject looks at an object at an infinite distance with both eyes, the eyeball 51L and the eyeball 51R face in the same visual direction. Because the left and right eyeballs follow Listing's law, the postures thereof after rotating are also the same. At this time, there is no difference between the retinal images of the left and right eyes.

On the other hand, as shown in FIG. 13B, if a subject looks at an object (a point A) at a finite distance, convergence is required. In this case, because the visual directions of the eyeball 51L and the eyeball 51R are different from each other, the amounts of rotation of the left and right eyeballs are different from each other. In FIG. 13B, the point A is located on the forward-left side. Therefore, the amount of rotation of the eyeball 51R is larger than the amount of rotation of the eyeball 51L.

Regarding the eyeball rotation based on Listing's law, the posture of the eyeball after rotation, that is, each of the direction vectors of the Y-axis and the Z-axis after rotation, depends on the visual direction vector indicated by the equation (1). If the visual direction vectors of the left eye and the right eye are different from each other, the direction vectors of the Y-axis and the Z-axis after rotation are different between the left and right eyes. Therefore, a rotational shift occurs in the retinal images. In order to cancel the rotational shift of the retinal images, rotation about the line of sight is required for the left and right eyes. Such rotation about this line of sight is fusional rotation.

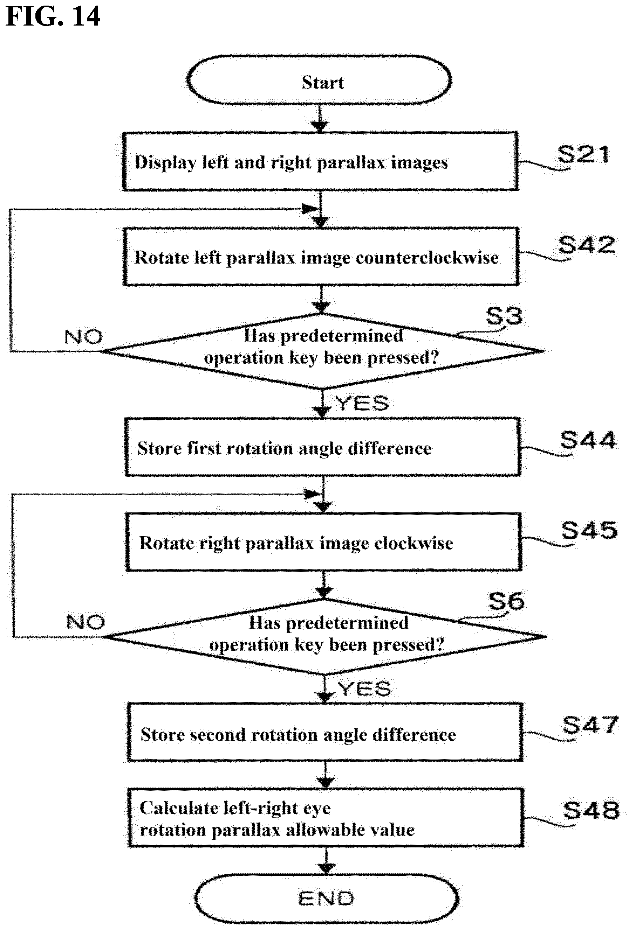

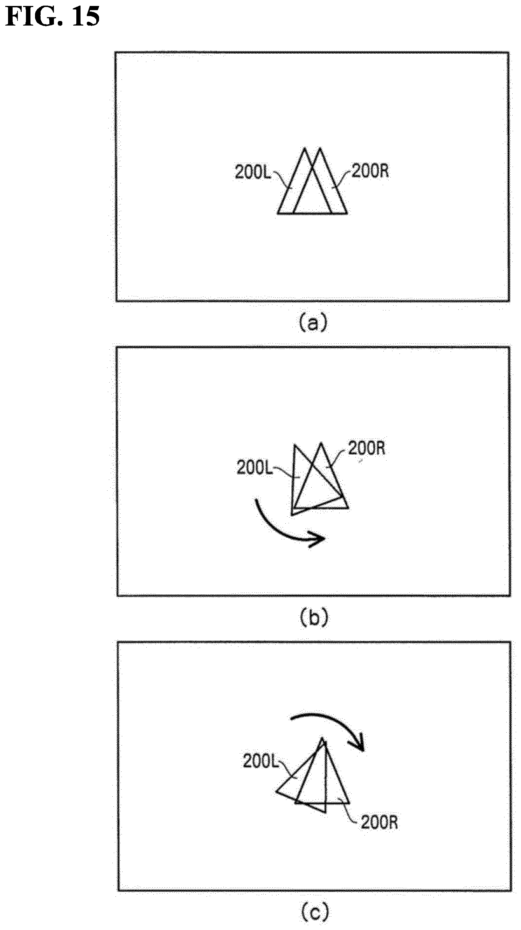

When fusional rotation occurs, rotation parallax arises between the left and right eyes. In the binocular visual function measurement program, it is possible to measure a left-right eye rotation parallax allowable value, which is an allowable value of rotation parallax of the left and right eyes that can enable stereoscopic vision. When the measurement item "left-right eye rotation parallax allowable value" is selected, the binocular visual function measurement program transitions to the left-right eye rotation parallax allowable value measurement mode in which the left-right eye rotation parallax allowable value of the measurement subject 2 is measured. FIG. 14 is a diagram showing a flowchart of processing executed by the binocular visual function measurement program in the left-right eye rotation parallax allowable value measurement mode. FIG. 15 shows transition diagrams of images displayed on the display screen during execution of the left-right eye rotation parallax allowable value measurement mode.

As shown in FIG. 15(b), when the binocular visual function measurement program transitions to the left-right eye rotation parallax allowable value measurement mode and images are displayed (S21 in FIG. 14, FIG. 15(a)), the left eye image 200L rotates counterclockwise (S42 in FIG. 14). The rotation of the left eye image 200L is rendered changing continuously or incrementally. The left eye image 200L continues to rotate until the predetermined operation key of the input device 138 is pressed (S42 and NO in S3 in FIG. 14). When the predetermined operation key is pressed by the measurement subject 2 (YES in S3 in FIG. 14), the rotation angle difference between the left eye image 200L and the right eye image 200R at this time (hereinafter referred to as a "first rotation angle difference" for convenience of description) is stored in the HDD 134 (S44 in FIG. 14). Note that the center of rotation of an image is defined as the center of mass of the image. In the left-right eye rotation parallax allowable value measurement mode, it is sufficient that the rotation angle difference between the left eye image 200L and the right eye image 200R changes relative to each other. Therefore, during measurement, the left eye image 200L and the right eye image 200R may be rotated simultaneously at different speeds or in different directions.

As shown in FIG. 15(c), in the processing of S45 in FIG. 14, the left eye image 200L rotates clockwise. When the predetermined operation key is pressed by the measurement subject 2 (YES in S6 in FIG. 14), the rotation angle difference between the left eye image 200L and the right eye image 200R at this time (hereinafter referred to as a "second rotation angle difference" for convenience of description) is stored in the HDD 134 (S47 in FIG. 14).

In the processing of S48 in FIG. 14, the left-right eye rotation parallax allowable value in the visual range is calculated based on the first and second rotation angle differences and the visual range. When measurement is performed in the left-right eye rotation parallax allowable value measurement mode while changing the visual range, the left-right eye rotation parallax allowable value when different accommodation occurs (e.g., when a subject looks at a near position or distant position) is measured. It is possible to optimally correct astigmatism by measuring the left-right eye rotation parallax allowable value for each of the different visual ranges and prescribing different astigmatism axes for the distance portion and the near portion, when prescribing a progressive refraction eyeglass lens, for example.

(Other measurements)

It is also possible to measure stereoscopic vision using the binocular visual function measurement system 10. In the measurement for stereoscopic vision, two types of parallax images having different shapes are displayed on the right eye image display panel 111R and the left eye image display panel 111L, for example. It is desirable that a parallax image has a simple geometrical shape such as a circle or a triangle such that the measurement subject 2 can focus on measurement. In the present embodiment, the two types of parallax images are a circle image and a triangle image. The circle image has a larger degree of parallax than the triangle image has. Therefore, the measurement subject 2 sees the circle image on the near side, and sees the triangle image on the far side. Then, the parallax of at least one of the circle image and the triangle image can be changed continuously or incrementally. The measurement subject 2 presses the predetermined operation key of the input device 138, for example, when the measurement subject 2 feels that neither of the circle image and the triangle image has depth or when the measurement subject 2 sees two images. The parallax of images obtained when the predetermined operation key is pressed is stored in the HDD 134. The CPU 132 calculates the limit to which the measurement subject 2 is able to perform stereoscopic viewing based on the stored parallax of images and visual range.

(Composite measurements of measurement items)

In each of the above-described various measurement modes, one measurement item is measured. In another measurement mode, a composite measurement may be performed in which composite measurement items (e.g., at least two of the convergence range, left-right eye vertical divergence allowable value, first unequal magnification allowable value, second unequal magnification allowable value, and left-right eye rotation parallax allowable value) are measured simultaneously. In particular, if a plurality of measurement items that are closely related to each other are measured simultaneously, the results of a measurement that cannot be recognized by the result of measurement of a single measurement item may be obtained. The operator can select any measurement items to be measured simultaneously. Some combinations of the measurement items may be prepared in advance. The following describes three examples of composite measurement.

(Composite measurement of convergence range - left-right eye vertical divergence allowable value)

The convergence range and the vertical divergence have strong mutual interaction, for example. In view of this, in the first composite measurement mode, the convergence range and the left-right eye vertical divergence allowable value are measured simultaneously by moving, continuously or incrementally, at least one of the left eye image 200L and the right eye image 200R in an oblique direction on the screen. Here, the "oblique direction on the screen" refers to all of the directions other than the horizontal direction on the screen or the vertical direction on the screen, and include a horizontal direction component on the screen and a vertical direction component on the screen. That is, a change of the display (hereinafter referred to as a "composite change" for convenience of description) obtained by combining change patterns (movement in the horizontal direction on the screen and movement in the vertical direction on the screen) in the convergence range measurement mode and in the left-right eye vertical divergence allowable value measurement mode is given to the left eye image 200L or the right eye image 200R. The angle of the oblique direction on the screen may be set by the operator or predetermined by the binocular visual function measurement program.

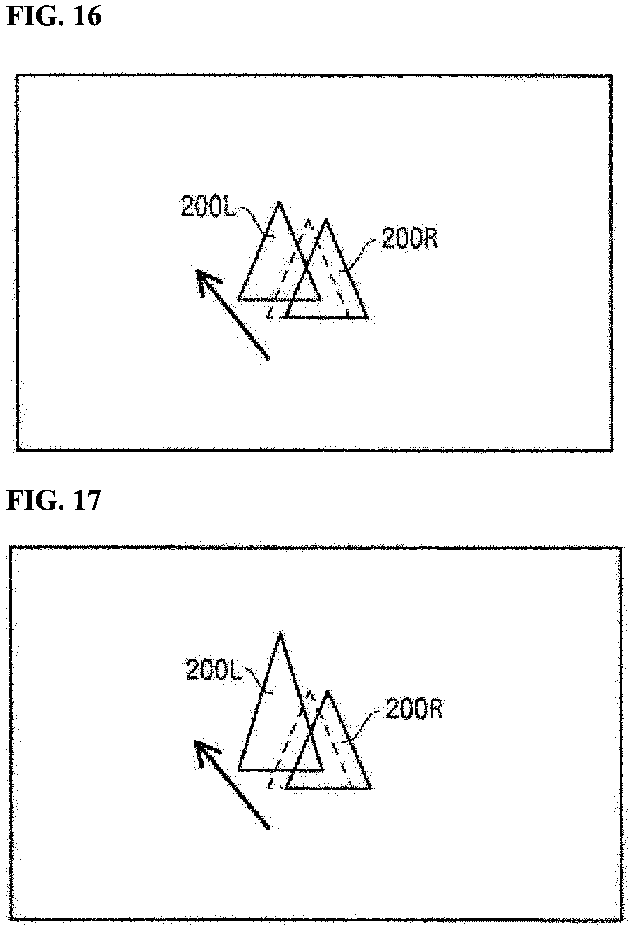

FIG. 16 shows an example of the display of the images in the first composite measurement mode. The flowchart of the first composite measurement mode is the same as the flowchart of the convergence range measurement mode or the like, and thus is omitted. According to the example in FIG. 16, the left eye image 200L moves in the oblique direction on the screen from a position indicated by the dashed line in FIG. 16. The movement in the oblique direction on the screen continues until the predetermined operation key of the input device 138 is pressed. When the predetermined operation key is pressed by the measurement subject 2, the amounts of positional shift of the left eye image 200L and the right eye image 200R at this time are stored in the HDD 134. In a sequence of measurement, a plurality of pieces of data regarding the amounts of positional shift may be collected while moving the left eye image 200L or the right eye image 200R in different oblique directions on the screen. The results of composite measurement of the convergence range and the left-right eye vertical divergence allowable value in the visual range are calculated based on the amounts of positional shift and the visual range stored in the HDD 134. When measurement is performed in the first composite measurement mode while changing the visual range, the results of composite measurement when different accommodation occurs (e.g., when a subject looks at a near position or distant position) are obtained.

(Composite measurement of convergence range - left-right eye vertical divergence allowable value - second unequal magnification allowable value (or first unequal magnification allowable value))

The convergence range, the vertical divergence, and unequal magnification of the left and right eyes have strong mutual interaction, for example. In view of this, in the second composite measurement mode, at least one of the left eye image 200L and the right eye image 200R is moved in the oblique direction on the screen continuously or incrementally, and is displayed in an enlarged or reduced size. If enlargement or reduction of an image is limited to a specific direction, the second unequal magnification allowable value is measured, whereas, if an image is enlarged or reduced in a fixed aspect ratio, the first unequal magnification allowable value is measured. A composite change, which is obtained by combining the changing patterns (movement in the horizontal direction on the screen, movement in the vertical direction on the screen, a change in display magnification) in the convergence range measurement mode, left-right eye vertical divergence allowable value measurement mode, or second unequal magnification allowable value (or first unequal magnification allowable value) measurement mode, is given to the left eye image 200L or the right eye image 200R. The ratio at which each change pattern is given may be set by the operator or may be predetermined by the binocular visual function measurement program.
FIG. 17 shows an example of the display of the images in the second composite measurement mode. The flowchart of the second composite measurement mode is the same as the flowchart of the convergence range measurement mode and the like, and thus is omitted. According to the example in FIG. 17, the left eye image 200L moves in the oblique direction on the screen from a position indicated by the dashed line in FIG. 17, and is enlarged only in the vertical direction on the screen. The left eye image 200L continues to be moved and enlarged until the predetermined operation key of the input device 138 is pressed. When the predetermined operation key is pressed by the measurement subject 2, the amounts of positional shift of the left eye image 200L and the right eye image 200R and the display magnification ratio in the vertical direction on the screen at this time (hereinafter referred to as an "image change state" for convenience of description) are stored in the HDD 134. In a sequence of measurement, a plurality of pieces of data regarding image change states may be collected while repeating movement and enlargement of the left eye image 200L or the right eye image 200R in different patterns. The results of composite measurement of the convergence range, the left-right eye vertical divergence allowable value, and the second unequal magnification allowable value (or the first unequal magnification allowable value) in the visual range are calculated based on the image change states and the visual range stored in the HDD 134. When measurement is performed in the second composite measurement mode while changing the visual range, the results of composite measurement when different accommodation occurs (e.g., when a subject looks at a near position or distant position) are obtained.

(Composite measurement of convergence range - left-right eye rotation parallax allowable value)

As described above, fusional rotation occurs accompanying convergence. In view of this, in the third composite measurement mode, at least one of the left eye image 200L and the right eye image 200R is moved in the horizontal direction on the screen continuously or incrementally, and is rotated clockwise or counterclockwise. That is, a composite change obtained by combining change patterns (movement in the horizontal direction on the screen and rotation about the center of mass of the image) in the convergence range measurement mode and in the left-right eye rotation parallax allowable value measurement mode is given to the left eye image 200L or the right eye image 200R. The ratio at which each change pattern is given may be set by the operator or may be predetermined by the binocular visual function measurement program.

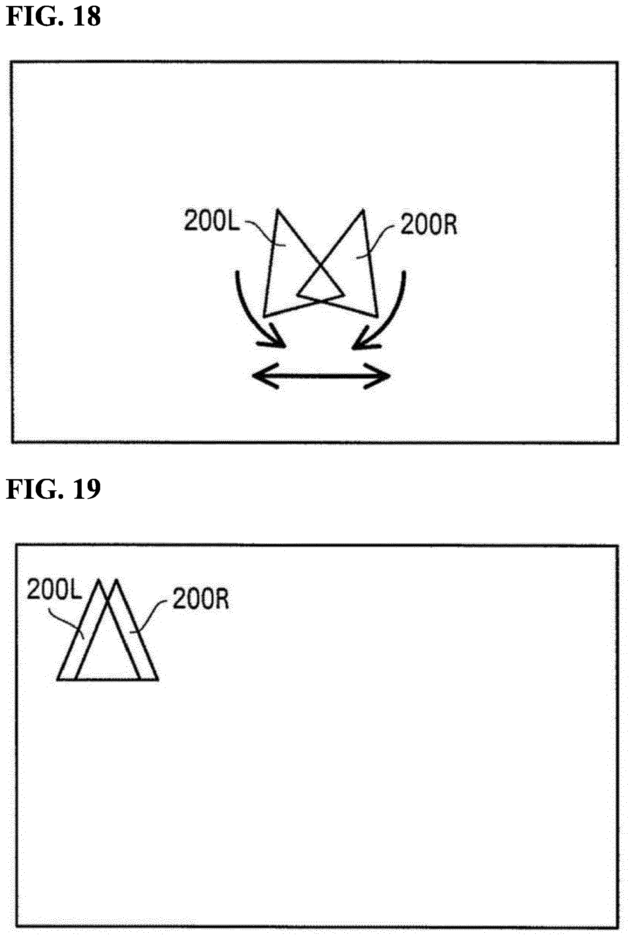

FIG. 18 shows an example of the display of the images in the third composite measurement mode. The flowchart of the third composite measurement mode is the same as the flowchart of the convergence range measurement mode and the like, and thus is omitted. According to the example in FIG. 18, the left eye image 200L and the right eye image 200R move in the horizontal direction on the screen and rotate counterclockwise and clockwise while separating from each other. The left eye image 200L and the right eye image 200R continue to be moved and rotated until the predetermined operation key of the input device 138 is pressed. When the predetermined operation key is pressed by the measurement subject 2, the amounts of positional shift of the left eye image 200L and the right eye image 200R and the rotation angle difference therebetween at this time are stored in the HDD 134. In a sequence of measurement, a plurality of pieces of data regarding the amounts of positional shift and the rotation angle difference may be collected while repeating movement and rotation of the left eye image 200L or the right eye image 200R in different patterns. The results of composite measurement of the convergence range and the left-right eye rotation parallax allowable value in the visual range are calculated based on the amounts of positional shift, the rotation angle difference, and the visual range stored in the HDD 134. When measurement is performed in the third composite measurement mode while changing the visual range, the results of composite measurement when different accommodation occurs (e.g., when a subject looks at a near position or distant position) are obtained.

(Measurement in consideration of lateral view)

In each of the above-described measurement modes, the left eye image 200L and the right eye image 200R are displayed in the center portion of the display screen. Therefore, this measurement only provides the results of measurement performed in a state where the measurement subject 2 faces forward. In view of this, after measurement in the state where the measurement subject 2 faces forward in each measurement mode is complete, as shown in FIG. 19, the positions where the left eye image 200L and the right eye image 200R are displayed are moved to a peripheral portion (the upper left corner of the screen) of the display screen, for example. Because the HMD 110 is worn on the head of the measurement subject 2 and the positional relationship therebetween is fixed, the left eye image 200L and the right eye image 200R are viewed from lateral sides. When measurement is performed in this state, the results of measurement in a state where the measurement subject 2 looks at the images from a lateral side are obtained. Furthermore, when measurement is performed while successively moving the left eye image 200L and the right eye image 200R to different positions in the peripheral portion on the screen (e.g., the center portion of the upper end of the screen, the upper right corner of the screen, the center portion of the right end of the screen, the lower right corner of the screen,...), the results of measurement in the lateral view state in various directions are obtained. A lateral view differs from a front view in conditions such as fusional rotation always being involved, for example. Therefore, results of measurement that are different from those in a front view are obtained. If an eyeglass lens is designed in consideration of such results of measurement, a more suitable prescription can be obtained.

<Effects of the Present Embodiment>

According to the present embodiment, one or more effects described below can be obtained.

(a) In the present embodiment, when the binocular visual function of the measurement subject 2 is measured, a right eye image and a left eye image (i.e., parallax images) are presented to the measurement subject 2 in a space where real space information is blocked out. Thus, the measurement subject 2 does not acquire information (real space information) that gives a sense of depth and perspective from the outside world, in addition to the presented parallax images. Also, the parallax images can be accurately positioned regardless of the direction of the face of the measurement subject 2, and there is no risk that an error will occur in the median plane. Therefore, according to the present embodiment, it is possible to precisely measure the capability relating to a pure binocular visual function without giving a sense of depth to the measurement subject 2. Furthermore, it is possible to very easily measure a binocular visual function without requiring a large-scale system configuration such as a stationary three-dimensional compatible video monitor. That is, according to the present embodiment, the binocular visual function of the measurement subject 2 can be very easily measured with high accuracy.
(b) In the present embodiment, the binocular visual function of a measurement subject is measured using the HMD 110 worn by the measurement subject 2. Thus, it is possible to easily and reliably present parallax images in a space where real space information is blocked out, thus suppressing installation costs therefor. Therefore, the realization of the presentation of parallax images thereon is preferable in order to very easily measure the binocular visual function with high accuracy.
(c) As described in the present embodiment, if values for specifying the convergence range of the measurement subject 2 are calculated as predetermined parameter values for the binocular visual function of the measurement subject 2, it is possible to very easily measure the convergence range of the measurement subject 2 with high accuracy. Also, by using the results of the above measurement as one of the parameters for designing an eyeglass lens, it is possible to provide an eyeglass lens suitable for the measurement subject 2.
(d) As described in the present embodiment, if the level of the ability of an eye of the measurement subject 2 to track a change in positions of the presented images is determined and the speed of a change in the positions of the presented images is then determined based on the results of the determination, the level of the tracking ability of the eye of the measurement subject 2 reflects on the speed of a change in the position of an image presented on the left side, and thus the measurement subject 2 can move his/her eyeballs without difficulty. As a result, it is possible to measure the binocular visual function of the measurement subject 2 with high accuracy.

<Variations and the like>

Although the embodiment of the present invention was described above, the disclosed content described above illustrates exemplary embodiments of the present invention. That is to say, the technical scope of the present invention is not limited to the above-described exemplary aspects, and various modifications can be made without departing from the gist thereof.

Although the case where the parallax images are presented using the HMD 110 worn by the measurement subject 2 in a space where real space information is blocked out has been described as an example in the above-described embodiment, the present invention is not limited to this, and a configuration may be adopted in which parallax images are presented using another means in a space where real space information is blocked out.

Also, the above-described embodiment was described on the premise that the moving speed, rotation speed, and scaling speed of the left eye image 200L or the right eye image 200R are kept constant, for example. However, the present invention is not limited to this, and movement, rotation, or scaling of the left eye image 200L or the right eye image 200R may be accelerated.

List of Reference Numerals

| | |
|---|---|
| 1 | Eyeglass lens manufacturing system |
| 10 | Binocular visual function measurement system |
| 20 | Input device |
| 30, 130 | PC |
| 40, 140 | Display |
| 50 | Processing device |
| 110 | HMD |
| 111R | Right eye image display panel |
| 111L | Left eye image display panel |
| 112R, 112L | Display optical system |
| 132 | CPU |
| 132a | Presentation control unit |
| 132b | Timing detection unit |
| 132c | Parameter value calculation unit |
| 132d | Tracking ability determination unit |
| 132e | Speed determination unit |
| 200R | Right eye image |
| 200L | Left eye image |

**Claims**

1. A binocular visual function measurement method comprising:

   a visual target presentation step of presenting a right eye image to be viewed by the right eye of a measurement subject and a left eye image to be viewed by the left eye of the measurement subject to the subject in a space where real space information is blocked out;
   a presentation control step of changing positions where the right eye image and the left eye image are presented, relative to each other;
   a timing detection step of detecting a timing at which the measurement subject is unable to fuse the right eye image and the left eye image when the presentation positions are changed; and
   a parameter value calculation step of calculating a predetermined parameter value regarding a binocular visual function of the measurement subject based on a relationship between the relative positions of the right eye image and the left eye image when the timing is detected.

2. The binocular visual function measurement method according to claim 1,
   wherein the right eye image and the left eye image are presented using a head mounted display to be worn by the measurement subject.

**3.** The binocular visual function measurement method according to claim 1 or 2,
wherein the predetermined parameter value is a value for specifying a convergence range of the measurement subject.

**4.** The binocular visual function measurement method according to any one of claims 1 to 3, further comprising

a tracking ability determination step of determining a level of the ability of an eye of the measurement subject to track a change in positions of the presented images,
wherein, in the presentation control step, the speed of a change in the relative positions of the right eye image and the left eye image is determined based on a result of the determination performed in the tracking ability determination step.

**5.** The binocular visual function measurement method according to any one of claims 1 to 4,
wherein the right eye image and the left eye image are constituted by figures having the same shape and the same size.

**6.** The binocular visual function measurement method according to any one of claims 1 to 5, further comprising
a visual range setting step of setting a visual range of the measurement subject with respect to the right eye image and the left eye image.

**7.** A binocular visual function measurement program for causing a computer to execute the binocular visual function measurement method according to any one of claims 1 to 6.

**8.** An eyeglass lens designing method comprising:

a step of measuring the binocular visual function of the measurement subject using the binocular visual function measurement method according to any one of claims 1 to 6; and
a step of determining an optical design value of the eyeglass lens based on a result of the measurement of the binocular visual function.

**9.** An eyeglass lens manufacturing method comprising:

a step of designing an eyeglass lens using the eyeglass lens designing method according to claim 8; and
a step of manufacturing the eyeglass lens according to a result of designing the eyeglass lens.

**10.** A binocular visual function measurement system comprising:

a visual target presentation unit configured to present a right eye image to be viewed by the right eye of a measurement subject and a left eye image to be viewed by the left eye of the measurement subject to the subject in a space where real space information is blocked out;
a presentation control unit configured to change positions where the right eye image and the left eye image are presented, relative to each other;
a timing detection unit configured to detect a timing at which the measurement subject is unable to fuse the right eye image and the left eye image when the presentation positions are changed; and
a parameter value calculation unit configured to calculate a predetermined parameter value regarding a binocular visual function of the measurement subject based on a relationship between the relative positions of the right eye image and the left eye image when the timing is detected.

**11.** The binocular visual function measurement system according to claim 10,
wherein the visual target presentation unit is configured using a head mounted display to be worn by the measurement subject.

## FIG. 1

10 = Binocular visual function
       measurement system

## FIG. 2

# FIG. 3

110

**HMD**

111L — Left eye image display panel | Right eye image display panel — 111R

130

**PC**

132 — **CPU**

132a — Presentation control unit

132b — Timing detection unit

132c — Parameter value calculation unit

132d — Tracking ability determination unit

132e — Speed determination unit

HDD — 134

RAM — 136

Input device — 138

140 — **Display**

# FIG. 4

```
        ┌─────────────┐
        │    Start    │
        └──────┬──────┘
               ↓
   ┌───────────────────────────────┐
   │ Display left and right parallax│  S1
   │ images                         │
   └───────────────┬───────────────┘
               ↓
   ┌───────────────────────────────┐
   │ Separate left and right        │  S2
   │ parallax images in direction   │
   │ of arrow A                     │
   └───────────────┬───────────────┘
               ↓
          Has predetermined          S3
   NO ◇  operation key been pressed? ◇
               ↓ YES
   ┌───────────────────────────────┐
   │ Store first relative           │  S4
   │ convergence measurement        │
   │ positions                      │
   └───────────────┬───────────────┘
               ↓
   ┌───────────────────────────────┐
   │                                │  S5
   └───────────────┬───────────────┘
               ↓
          Has predetermined          S6
   NO ◇  operation key been pressed? ◇
               ↓ YES
   ┌───────────────────────────────┐
   │ Store second relative          │  S7
   │ convergence measurement        │
   │ positions                      │
   └───────────────┬───────────────┘
               ↓
   ┌───────────────────────────────┐
   │ Calculate positive relative    │  S8
   │ convergence and negative       │
   │ relative convergence           │
   └───────────────┬───────────────┘
               ↓
        ┌─────────────┐
        │     END     │
        └─────────────┘
```

S5 = Bring or separate left and right parallax images
closer to/from each other in direction of arrow B

# FIG. 5

(a)

(b)

(c)

## FIG. 6

```
        ┌─────────────────────────┐
        │          Start          │
        └─────────────────────────┘
                     │
                     ▼
   ┌──────────────────────────────────┐
   │ Display left and right parallax  │   S1
   │ images                           │
   └──────────────────────────────────┘
                     │
                     ▼
   ┌──────────────────────────────────┐
   │ Separate left and right parallax │   S12
   │ images from each other in        │
   │ direction of arrow C             │
   └──────────────────────────────────┘
                     │
                     ▼
        ╱────────────────────────╲
  NO   ╱   Has predetermined       ╲   S3
◄──────   operation key been pressed?
        ╲                          ╱
         ╲────────────────────────╱
                     │ YES
                     ▼
   ┌──────────────────────────────────┐
   │ Store first left-right eye       │   S14
   │ vertical divergence allowable    │
   │ value measurement positions      │
   └──────────────────────────────────┘
                     │
                     ▼
   ┌──────────────────────────────────┐
   │                                  │   S15
   └──────────────────────────────────┘
                     │
                     ▼
        ╱────────────────────────╲
  NO   ╱   Has predetermined       ╲   S6
◄──────   operation key been pressed?
        ╲                          ╱
         ╲────────────────────────╱
                     │ YES
                     ▼
   ┌──────────────────────────────────┐
   │ Store second left-right eye      │   S17
   │ vertical divergence allowable    │
   │ value measurement positions      │
   └──────────────────────────────────┘
                     │
                     ▼
   ┌──────────────────────────────────┐
   │ Calculate left-right eye         │   S18
   │ vertical divergence allowable    │
   │ value                            │
   └──────────────────────────────────┘
                     │
                     ▼
        ┌─────────────────────────┐
        │           END           │
        └─────────────────────────┘
```

S15 = Bring or separate left and right parallax images
       closer to/from each other in direction of arrow D

# FIG. 7

(a)

(b)

(c)

# FIG. 8

```
                    ( Start )
                        │
                        ▼
┌──────────────────────────────────────┐
│ Display left and right parallax images│  S21
└──────────────────────────────────────┘
                        │
                        ▼
┌──────────────────────────────────────┐
│ Enlarge left parallax image           │  S22
└──────────────────────────────────────┘
                        │
                        ▼
         ╱ Has predetermined ╲              S3
 NO ────<  operation key been pressed? >
         ╲                    ╱
                        │ YES
                        ▼
┌──────────────────────────────────────┐
│ Store first display magnification ratio│  S24
└──────────────────────────────────────┘
                        │
                        ▼
┌──────────────────────────────────────┐
│ Enlarge right parallax image          │  S25
└──────────────────────────────────────┘
                        │
                        ▼
         ╱ Has predetermined ╲              S6
 NO ────<  operation key been pressed? >
         ╲                    ╱
                        │ YES
                        ▼
┌──────────────────────────────────────┐
│ Store second display magnification ratio│ S27
└──────────────────────────────────────┘
                        │
                        ▼
┌──────────────────────────────────────┐
│ Calculate first unequal magnification │  S28
│ allowable value                       │
└──────────────────────────────────────┘
                        │
                        ▼
                    ( END )
```

# FIG. 9

(a)

(b)

(c)

# FIG. 10

```
                    ┌──────────────┐
                    │    Start     │
                    └──────┬───────┘
                           │
                           ▼
         ┌─────────────────────────────┐
         │ Display left and right      │  S21
         │ parallax images             │
         └──────────────┬──────────────┘
                        │
                        ▼
         ┌─────────────────────────────┐
         │ Enlarge left parallax image │  S32
         │ only in specific direction  │
         └──────────────┬──────────────┘
                        │
                        ▼
                ◇ Has predetermined          S3
           NO ◇  operation key been pressed? ◇
                        │ YES
                        ▼
         ┌─────────────────────────────┐
         │                             │  S34
         └──────────────┬──────────────┘
                        │
                        ▼
             ◇ Has enlargement              S35
            ◇ processing been performed  ◇  YES
             ◇ in all specific directions? ◇
                        │ NO
                        ▼
         ┌──────────────────────┐   ┌──────────────────────────┐
         │ Change specific      │S36│ Calculate second unequal │ S38
         │ direction of         │   │ magnification allowable  │
         │ scaling target       │   │ value                    │
         └──────────────────────┘   └────────────┬─────────────┘
                                                 ▼
                                          ┌─────────────┐
                                          │     END     │
                                          └─────────────┘
```

S34 = Store display magnification
ratio in specific direction in
which image is enlarged

# FIG. 11

(a)

(b)

(c)

**FIG. 12**

**FIG. 13**

A                              B

## FIG. 14

Start

Display left and right parallax images — S21

Rotate left parallax image counterclockwise — S42

Has predetermined operation key been pressed? — S3
NO / YES

Store first rotation angle difference — S44

Rotate right parallax image clockwise — S45

Has predetermined operation key been pressed? — S6
NO / YES

Store second rotation angle difference — S47

Calculate left-right eye rotation parallax allowable value — S48

END

**FIG. 15**

(a)

(b)

(c)

## FIG. 16

## FIG. 17

**FIG. 18**

**FIG. 19**

## INTERNATIONAL SEARCH REPORT

| International application No. |
| --- |
| PCT/JP2020/028743 |

**A. CLASSIFICATION OF SUBJECT MATTER**
Int.Cl. G02C7/02(2006.01)i, A61B3/08(2006.01)i, G02B27/02(2006.01)i
FI: A61B3/08, G02C7/02, G02B27/02Z

According to International Patent Classification (IPC) or to both national classification and IPC

**B. FIELDS SEARCHED**

Minimum documentation searched (classification system followed by classification symbols)
Int.Cl. G02C7/02, A61B3/08, G02B27/02

Documentation searched other than minimum documentation to the extent that such documents are included in the fields searched
Published examined utility model applications of Japan    1922-1996
Published unexamined utility model applications of Japan    1971-2020
Registered utility model specifications of Japan    1996-2020
Published registered utility model applications of Japan    1994-2020

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| Y | JP 2005-524432 A (THE UNIVERSITY OF NOTTINGHAM) 18.08.2005 (2005-08-18), paragraphs [0001]-[0177] | 1-11 |
| Y | JP 2012-95693 A (HOYA CORPORATION) 24.05.2012 (2012-05-24), paragraphs [0001]-[0093] | 1-11 |

☐ Further documents are listed in the continuation of Box C.    ☒ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 28.09.2020 | 06.10.2020 |

| Name and mailing address of the ISA/ | Authorized officer |
| --- | --- |
| Japan Patent Office 3-4-3, Kasumigaseki, Chiyoda-ku, Tokyo 100-8915, Japan | Telephone No. |

Form PCT/ISA/210 (second sheet) (January 2015)

INTERNATIONAL SEARCH REPORT
Information on patent family members

International application No.

PCT/JP2020/028743

| | | |
|---|---|---|
| JP 2005-524432 A | 18.08.2005 | US 2006/0087618 A1 paragraphs [0001]-[0228] WO 2003/092482 A1 |
| JP 2012-95693 A | 24.05.2012 | US 2012/0105609 A1 paragraphs [0001]-[0111] DE 102011054833 A1 |

Form PCT/ISA/210 (patent family annex) (January 2015)

## REFERENCES CITED IN THE DESCRIPTION

**Patent documents cited in the description**

- JP 2012095693 A

- WO 2010090144 A

**Non-patent literature cited in the description**

- **SHINICHI SHIKANO.** Little Pupil Science. Kanehara & Co. Ltd, 1925, 50

- **TOYOHIKO HATADA.** Depth Information and Characteristics of Vision. Visual Information Research Group, 23 April 1974, 12